# EUROPEAN PATENT SPECIFICATION

(11) **EP 3 566 664 B1**
(45) Date of publication and mention of the grant of the patent: **02.03.2022**
(21) Application number: 18736597.8
(22) Date of filing: 02.01.2018
(51) Int. Cl.: A61B 17/29

(54) **SURGICAL TOOL**
CHIRURGISCHES INSTRUMENT
INSTRUMENT CHIRURGICAL

(30) Priority: 05.01.2017 ES 201730009
(43) Date of publication of application: 13.11.2019
(73) Proprietor: Universitat Politècnica de València, 46022 Valencia (ES); Fundación Para la Investigación del Hospital Universitario y Politécnico La Fe de la Comunidad Valenciana, 46026 Valencia (ES)
(72) Inventor: PACE BEDETTI, Horacio Martín, 46022 Valencia (ES); CONEJERO RODILLA, Andrés, 46022 Valencia (ES); MARTÍNEZ DE JUAN, José Luis, 46022 Valencia (ES); DOLZ LAGO, José Francisco, 46026 Valencia (ES)
(74) Representative: Maslanka Kubik, Dorota Irena
(86) International application number: PCT/ES2018/070001
(87) International publication number: WO 2018/127615

(56) References cited:
- WO-A1-2015/151093
- WO-A1-2016/125574
- WO-A2-2008/020964
- WO-A2-2008/020964
- ES-T3- 2 547 001
- MX-A- 2016 002 222
- US-A1- 2012 150 155
- US-A1- 2012 245 567
- US-B1- 6 605 105
- US-B2- 7 615 067

## Description

### Field of the Invention

The present invention generally relates to the field of surgery, and more specifically to the field of surgical tools to be applied in minimally invasive surgery techniques.

### Background of the Invention

For the surgical field, minimally invasive surgery (MIS or MAS) represents one of the most important breakthroughs over the past decades. Although it started out being used for gynaecological treatments, it has spread to other surgery disciplines, in part due to the advantages it offers the patient: less post-operative pain, shorter recovery times, lower risk of infection, and fewer marks left on the body, etc.

One of the areas that have benefitted the most from processes of this type is surgery performed in the pelvic-abdominal cavity, known as laparoscopic surgery or laparoscopy.

Surgical tools for laparoscopy and other types of minimally invasive surgeries known today in the art generally have scissor-type handles in the form of rings (see, for example, patent document US5782859) or pistol. In tools of this type, the surgeon inserts their fingers in the rings of the handle and acts on the rings to actuate the tip of the surgical tool (for example, a clamp). The tool disclosed in patent document US5782859 also includes a turning wheel or rotatable knob which allows rotating the position of the tip of the tool with respect to the handle in order to adopt the position required for the surgery.

In practice, however, despite the existence of said rotatable knob, after several hours of practical training, surgeons find it difficult to actuate this knob and as a result pronate their forearm in order to achieve the desired turning of the tip. They must then hold this uncomfortable position at all times until they finish whatever action they are performing.

Therefore, despite the fact that laparoscopic surgery features those advantages characteristic of MIS, in practice, surgeons must make certain concessions. This practice requires greater effort, concentration, and mental stress (Berguer, R., 1998. Surgical technology and the ergonomics of laparoscopic instruments, SurgEndosc, 462; Patkin, M., Isabel, L., 1995. Ergonomics, engineering and surgery of endosurgical dissection, Journal of the Royal College of Surgeons of Edinburgh 40, 132; Berguer, R., Smith, W.D., Chung, Y.H., 2001. Performing laparoscopic surgery is significantly more stressful for the surgeon than open surgery, SurgEndosc 15, 1204-1207).

As discussed above, during surgery surgeons have to adopt awkward and uncomfortable postures with their fingers, hands, wrists, and arms (Trejo, A., Jung, M., Oleynikov, D., Hallbeck, M.S., 2007. Effect of handle design and target location on insertion and aim with a laparoscopic surgical tool, Appl Ergon 38, 753) and these postures increase physical fatigue for the surgeon, causing tremors and potential errors that may result in the patient being injured during the operation.

These uncomfortable postures also affect the surgeon after the operation. In fact, one of the biggest causes of post-operative pain and numbness for surgeons is attributed to the postures adopted during laparoscopic surgery (Emam, T.A., Frank, T.G., Hanna, G.B., Cuschieri, A., 2001. Influence of handle design on the surgeon's upper limb movements, muscle recruitment, and fatigue during endoscopic suturing. SurgEndosc 15, 672; B Berguer, R., Forkey, D.L., Smith, W.D., 1999. Ergonomic problems associated with laparoscopic surgery. Surg Endosc 13, 466-8; Berguer, R., Gerber, S., Kilpatrick, G., Beckley, D., 1998. An ergonomic comparison of in-line vs pistol-grip handle configuration in a laparoscopic grasper. Surg Endosc 12, 805-8).

The existing literature asserts that the current handle design in the form of a ring of conventional surgical tools for minimally invasive surgery is the primary cause of these problems due to said handles poorly adapting to the surgeon's needs (Matern, U., Kuttler, G., Giebmeyer, C., Waller, P., Faist, M., 2004. Ergonomic aspects of five different types of laparoscopic instrument handles under dynamic conditions with respect to specific laparoscopic tasks: an electromyographic-based study. SurgEndosc 18, 1231-41).

Patent document US6666854B1 discloses an endoscopic surgical instrument which can incorporate, between the handle and the elongated shaft of the instrument, a ball joint-type element. This ball joint-type element provides more degrees of freedom to the movement of the tip of the tool, which allows the surgeon to have greater control over the movement of the tool in the abdominal cavity. However, it neither addresses nor solves the aforementioned problems for surgeons resulting from the uncomfortable movements and the postures they have to hold throughout the entire surgical intervention.

Similarly, WO 2015/151093 discloses a medical device. The medical device includes an elongated device body having a steerable portion including a plurality of segments. The segments are co-axially mounted over at least one elongated elastic element which is configured for limiting rotation of the segments with respect to each other. The medical device includes a control wire running alongside the elongated device body and being unrestrained at the steerable portion such that tensioning of the control wire angles the steerable portion from a longitudinal axis of the elongated device body and deflects the control wire away from the steerable portion. Although the device allows the surgeon to have greater control over the movement of the tool, it neither addresses nor solves the aforementioned problems for surgeons resulting from the uncomfortable movements and the postures they have to hold throughout the entire surgical intervention.

Robotic systems which allow performing surgical interventions without the surgeon having to the present in the operating room, thereby preventing the problems resulting from the surgeon's effort and exhaustion, are also known in the art. An example of this type of technology is the da Vinci^{®} surgical system. Systems of this type mark an enormous breakthrough because they provide greater precision and more degrees of freedom of movement than a human hand does. However, their cost is too high so most hospitals cannot afford them.

Therefore, there is still a need in the art for a new surgical tool for minimally invasive surgery which allows easing the effort and exhaustion interventions of this type represent for the surgeon. Furthermore, there is a need for a tool of this type that can be easily manufactured and therefore has a lower cost.

### Summary of the Invention

To solve the problems in the prior art, the present invention discloses a surgical tool comprising:
- a stylet;
- a tip of the surgical tool at the distal end of the stylet; and
- a handle at the proximal end of the stylet, the handle comprising means for actuating the tip of the tool;

Furthermore, the surgical tool disclosed in the present invention is characterised in that the handle is formed by a ball joint comprising:
- a base hemisphere coupled to the stylet;
- a central hemisphere solidly connected to the base hemisphere and located at the centre thereof;
- a gripping clamp with a spherical inner surface concentric with the base hemisphere;
- a rotation hemisphere solidly connected to the gripping clamp and located at the centre of the inner surface thereof, forming a joint of the ball-socket type together with the central hemisphere, such that the base hemisphere and the gripping clamp can turn with respect to one another; and
- locking means which allow a locking position to be adopted, in which relative movement between the gripping clamp and the base hemisphere is prevented, and an unlocking position, in which relative movement between the gripping clamp and the base hemisphere is permitted, wherein said locking means are formed by the gripping clamp itself, the locking area of which is formed by the spherical inner surface thereof, which can move until making contact with the base hemisphere by means of pressure on the sides of the gripping clamp.

As will be described herein below, as a result of the ball joint the surgeon can vary the working angle of the tool without this implying having to adopt an uncomfortable working position. Upon actuating the locking means, the surgeon can take the tip of the surgical tool to the required position to perform the intervention. Once the locking means are released, the surgeon can recover a more comfortable hand position that does not entail any effort and exhaustion, without said movement being transmitted to the rest of the tool and therefore to the tip of the tool.

### Brief Description of the Drawings

The present invention will be better understood in reference to the following drawings which illustrate a preferred embodiment of the invention, provided by way of example, and should not be interpreted as being limiting of the invention in any way.
Figure 1 shows a perspective view of a surgical tool according to the preferred embodiment of the present invention.
Figure 2 shows a vertical longitudinal section view of the handle of the tool of Figure 1.
Figure 3 shows a horizontal longitudinal section view of the handle of the tool of Figure 1.
Figure 4 shows a series of movements a surgeon makes to change the position of the tool of the present invention.
Figure 5 shows the postures a surgeon adopts to perform an intervention with several tools of the prior art as well as with the tool of the present invention.
Figures 6 to 8 show the results of a movement analysis study for analyzing the movements made by surgeons using a surgical tool of the prior art and a surgical tool according to the present invention.
Figure 9 is a graph showing the results of a surface electromyography study comparing a tool of the prior art and a surgical tool according to the present invention.

### Detailed Description of the Preferred Embodiments

As it is used herein, and as can be seen based on the attached drawings, the term "hemisphere" must be interpreted in a broad manner to include a spherical body that is not entirely closed forming complete sphere. It is not limited to a body which defines exactly half a sphere, but rather it may encompass more than or less than half a sphere.

Throughout the present document, the term "postural freedom" refers to the ease with which the surgeon can adopt the most beneficial positions during the operation, outside the abdominal cavity of the patient. While the "degrees of freedom" provide the surgeon with greater control over the work within the abdominal cavity, "postural freedom" allows the surgeon to move around outside the abdominal cavity, holding the standby position that is most beneficial to their musculature.

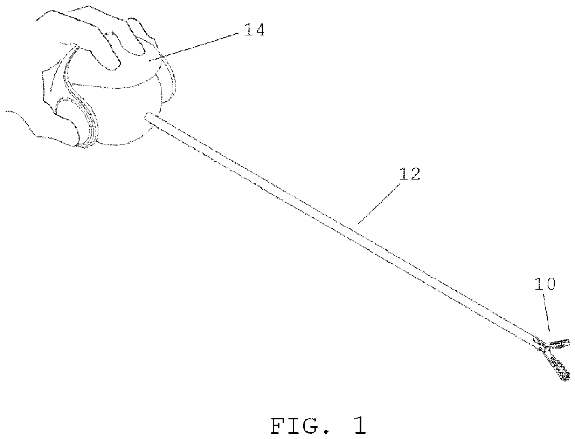

As can be observed in Figure 1, the surgical tool according to the preferred embodiment of the present invention comprises a tip (10) of the tool, a stylet (12), and a handle (14). The tip (10) of the tool is located at the distal end of the stylet (12), while the handle (14) is located at its proximal end.

Throughout the present document, the terms "distal" and "proximal" refer to locations with respect to a surgeon while using the tool. Thus, for example, the "distal" end of the stylet is the end farthest away from the surgeon, while the "proximal" end is the closest to the surgeon, when the surgeon holds the tool by the handle thereof.

In the embodiment shown in the drawings, the tip (10) of the tool is formed by laparoscopic clamps, however one skilled in the art will readily understand that the present invention can be applied to tools with different tips, whether they are for laparoscopy (for gripping, dissecting, suturing, and cutting) as tips suitable for endoscopy or high-frequency technology for cauterising, suturing, or cutting during the operation (adding in the latter case a one-pole or two-pole electrical outlet in a manner that is known in the art) or any other tip that may be included.

The stylet (12) is made up of an internal rod (16) and an outer sheath (18) as will be seen herein below. The internal rod (16) can move with respect to the outer sheath (18) such that it allows the actuation (opening and closing) of the tip (10) of the tool.

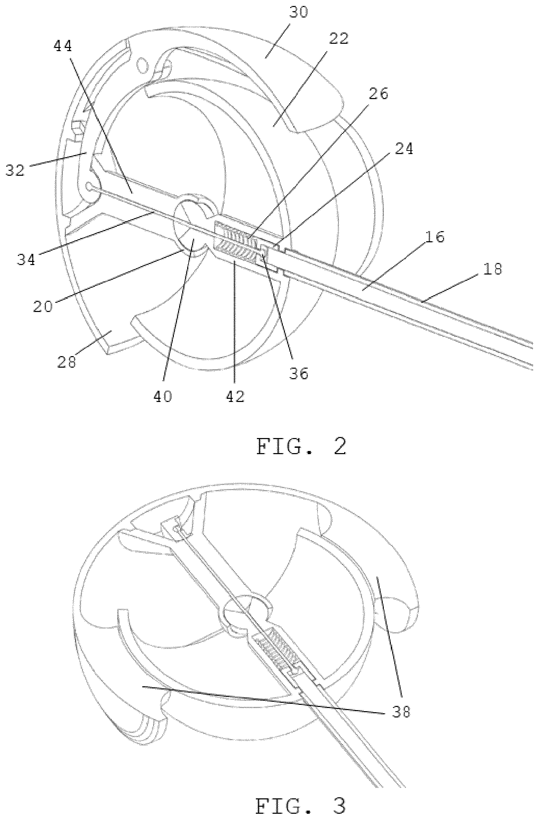

As can be seen in Figures 2 and 3, the handle (14) is formed by a ball joint comprising a base hemisphere (22), a central hemisphere (40) solidly connected to the base hemisphere and located at the centre thereof, a gripping clamp (28) with a spherical inner surface concentric with the base hemisphere, and a rotation hemisphere (20) solidly connected to the gripping clamp and located at the centre thereof.

The rotation hemisphere (20) and the central hemisphere (40) make up a joint of the ball-socket type, such that the gripping clamp (28) and the base hemisphere (22) can turn with respect to one another.

According to the preferred embodiment of the present invention, the central hemisphere (40) is solidly attached to the base hemisphere (22) by means of a base intermediate body (42), aligned with the stylet (12). The rotation hemisphere (20) is solidly attached to the gripping clamp (28) by means of an intermediate gripping body (44), aligned with the stylet (12) in the surgical tool neutral position. The surgical tool neutral position is that in which the gripping clamp (28) is not angularly moved with respect to the stylet (12).

According to the preferred embodiment of the present invention, the inner surface of the gripping clamp (28) is located facing the base hemisphere (22), for the purpose of preventing the base hemisphere (22) from running into the intermediate gripping body (28) to the greatest extent possible, thereby maximising the range of turning of the gripping clamp (28) around the base hemisphere (22).

The base hemisphere (22) is coupled such that it is solidly connected to the outer sheath (18) of the stylet (12). The base hemisphere (22) comprises therein the base intermediate body (42) having a longitudinal recess which allows the movement of a cable-rod connector (24) longitudinally along the inside of said recess and prevents the movement of the connector transverse to said recess. Furthermore, inside the hollow cylindrical element there are housed elastic return means implemented in this case by a spring (26). Therefore, these elements form a single block which will turn together at all times.

In turn, the gripping clamp (28) is coupled such that it is solidly connected to an actuation trigger (30). The trigger (30) is connected to an actuation lever (32), in turn connected to a proximal end of a cable (34) connected to a proximal end of the internal rod (16) of the stylet (12) .

As a result of the joint of the ball-socket type, the surgeon can vary the working angle of the tool without this implying having to adopt an uncomfortable working position.

The trigger (30), lever (32), cable (34), internal rod (16), and spring (26) assembly constitutes means for actuating the tip (10) of the tool.

Thus, when a surgeon actuates the trigger (30), this actuation is transmitted (through the lever (32), the cable (34), and the internal rod (16)) to the tip (10) of the tool. The tip (10) of the tool thereby transitions from a first (for example, open) position to a second (for example, closed) position. When the surgeon stops performing any action on the trigger (30), the spring (26) causes the internal rod (16) to return to its initial position, so the tip (10) of the tool returns from the second (for example, closed) position to the first (for example, open) position.

According to the preferred embodiment shown in Figure 2, actuation of the trigger (30) is transmitted to the lever (32) by means of a pivoting movement about an axis.

The aforementioned cable-rod connector (24) is attached to a cable stop (36), such that the free rotation of one element on the other while the translational movement is being transmitted is allowed.

It should be pointed out that according to the preferred embodiment of the present invention, both the outer sheath (18) and the internal rod (16) are coupled to the base hemisphere (22) and to the cable (34), respectively, according to removable attachment means which can be, for example, threaded attachments, stud bolts, intermediate parts, etc. The outer sheath (18) and the internal rod (16) together with the tip (10) of the tool can thereby be removed from handle of the tool. Different types of tips (10) of the tool can therefore be coupled to the surgical tool depending on the operation needs or they can be replaced with other new tips due to wear, malfunction, breakage, etc., without having to dispose of the rest of the tool.

As can be seen best in Figure 3, the handle (14) of the surgical tool also comprises locking means. According to the invention, these locking means are formed by the gripping clamp (28) itself, the locking area (38) of which is formed by the spherical inner surface thereof, which can move until making contact with the base hemisphere (22) by means of pressure on the sides of the gripping clamp (28).

According to another preferred embodiment, the locking area (38) is formed by a pair of locking projections arranged in the gripping clamp (28) in a space between said gripping clamp (28) and the base hemisphere (22). Each locking projection is arranged on an opposite side with respect to a central axis of the hemispheres (20, 22) defined by the longitudinal axis of the stylet (12).

Thus, when the surgeon applies pressure on the locking area (38), the pressure is transmitted to the base hemisphere (22) and a locking position is adopted in which relative movement of the gripping clamp (28) and the base hemisphere (22) is prevented. In this situation, the entire surgical tool moves as a whole and the surgeon can place the tip (10) of the tool in the suitable position to perform the intervention. Likewise, in the locking position the surgeon can actuate the tip (10) of the tool to grip and move tissue or an organ in the abdominal cavity over the course of the intervention. Upon releasing said pressure, the tool transitions to an unlocking position in which relative movement of the gripping clamp (28) with respect to the base hemisphere (22) is permitted again. Therefore, the surgeon can search for a position that is more comfortable for them and generate less effort and exhaustion without said movement being transmitted to the rest of the tool and therefore to the tip (10) of the tool.

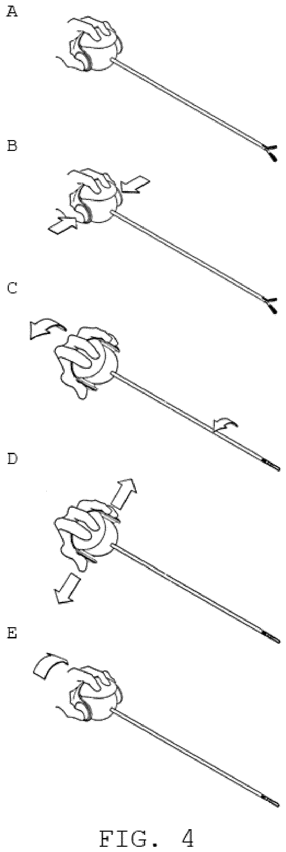

Figure 4 illustrates these movements by the surgeon. Figure 4-A illustrates a standby position in which the surgeon is holding the tool in a position that is comfortable for them. To change the position of the tip of the tool, the surgeon performs the series of movements illustrated in Figures 4-B to 4-E. First, as shown in Figure 4-B, the surgeon applies pressure on the sides of the ball joint constituting the handle, whereby actuating the locking area in order to arrange it in the locking position, as mentioned above. Once in said position, the surgeon performs a turn with their wrist (Figure 4-C) until the tip of the tool is in the suitable position. As mentioned above, since the locking area is in the locking position, the entire tool is integrally attached, and therefore the movements the surgeon makes with their hand are transmitted to the tip of the tool.

Next (Figure 4-D), the surgeon stops applying pressure on the sides of the ball joint, whereby releasing the locking area. As mentioned above, in this unlocking position relative movement of the rotation hemisphere with respect to the base hemisphere is permitted. Therefore, the surgeon can turn their wrist (Figure 4-E) again to a position that is more comfortable, while the rest of the tool (and specifically the tip of the tool) remains in the previous suitable operating position.

Therefore, as a result of the surgical tool of the present invention, the surgeon only needs to make isolated movements (for example, turns of the wrist) to place the tool in the correct position to perform an intervention. However, once the tool is located in said position, the surgeon can return to a position that is comfortable to them, thereby reducing the effort and fatigue the surgeon experiences. The tool according to the present invention therefore provides the surgeon with greater "postural freedom" outside the abdominal cavity, which none of the tools known in the state of the art allows, as they seek to provide more "degrees of freedom" in the abdominal cavity. This preferred embodiment has the advantage of the contact surface in the joint of the ball-socket type being very small, so friction during rotation of one hemisphere with respect to the other is reduced, thus optimising the operation of the surgical tool.

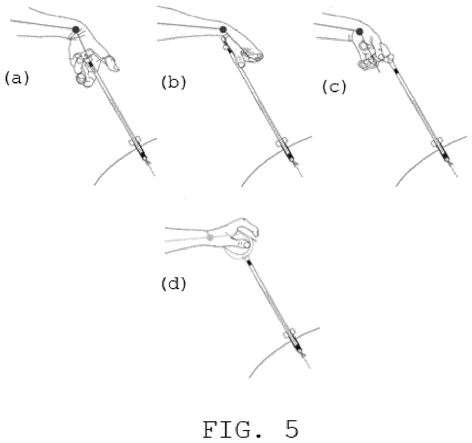

Figure 5 shows the posture a surgeon must hold with their arm and hand to hold a surgical tool in one and the same operating position with three tools of the prior art (a-c) as well as with a tool like the one described above according to the preferred embodiment of the present invention (d). As can be seen, in the case of the tools of the prior art (Figure 5, a-c), the surgeon must hold an unnatural and uncomfortable position, which will entail greater fatigue which may translate into tremors and involuntary movements (which can obviously affect the patient) as well as subsequent consequences for the surgeon. However, the tool according to the present invention (Figure 5d) allows the surgeon to hold a natural posture while the tool is in the intervention position as in the preceding cases.

Two studies were conducted to check the effect of a tool according to the present invention on the movements and efforts made by a surgeon during an intervention. These studies compared a conventional surgical tool of the prior art with a scissor-type handle with a tool according to the present invention with a ball joint-type handle like the one described above herein.

The first study consisted of a movement analysis performed at Hospital La Fe (Valencia). The movement analysis is a validated method commonly used for evaluating surgical skills. There are different studies providing movement analysis data for evaluating positions and movements of surgeons in an operating room.

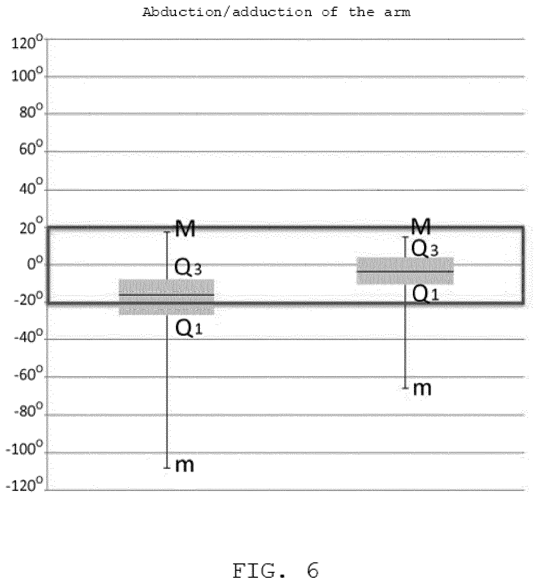

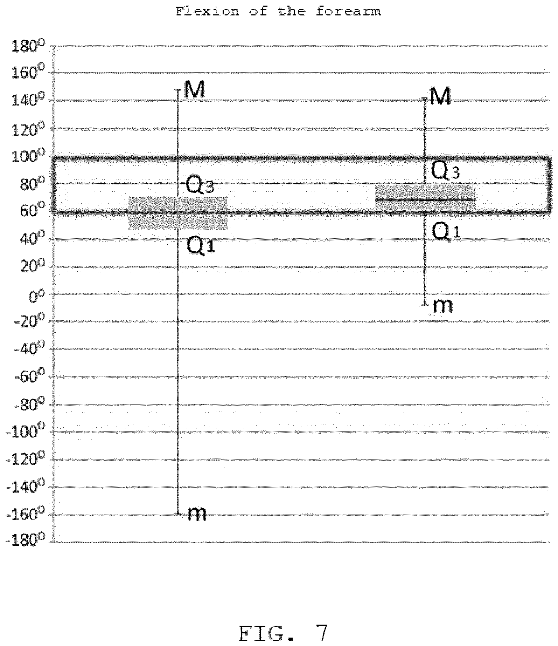

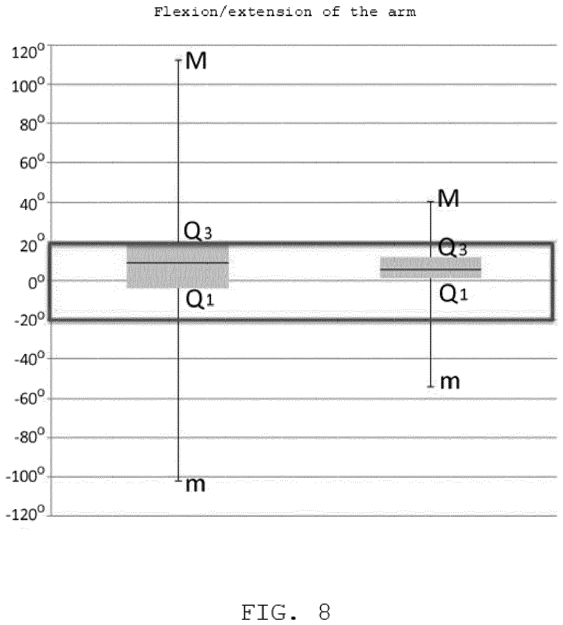

The study was conducted with surgeons and surgical residents comparing the aforementioned tools, and showed an improvement in the amount of neutral positions (risk-free) achieved by the surgeon: 34.9% in abduction/adduction of the arm (Figure 6), 30.6% in flexion of the forearm (Figure 7), and 21% in flexion/extension of the arm (Figure 8). In the graphs in Figures 6, 7, and 8, the left side represents the results for the tool of the prior art and the right side represents the results for the tool according to the present invention. The rectangle included in said figures represents the recommended range for the positions (risk-free) of the arm and forearm of the surgeon during use of the surgical tool: -20°/20° for the abduction/adduction of the arm (Figure 6), 60°/100° for the flexion of the forearm (Figure 7), and -20°/20° for the flexion/extension of the arm (Figure 8). Q₁ and Q₃ are the lower quartile and the upper quartile, respectively, which define the interquartile range of the data obtained in the study. M corresponds to the maximum value of the data and m corresponds to the minimum value of the data. The horizontal line located in the area representing the interquartile range is the statistical mean.

These results of the movement analysis study were evaluated by means of the Rapid Entire Body Assessment (REBA) method, proposed by Hignett and McAtamney (Hignett, S., McAtamney, L., 2000. Rapid entire body assessment (REBA). Appl Ergon 31, 201-5). This method allows analysing the positions adopted by the upper limbs, defining possible musculoskeletal risks and considering critical positions during the task.

The second study that was conducted was a comparative study of the muscular activity by means of surface electromyography (sEMG). This is a non-invasive study which allows evaluating and recording the electrical activity produced by the muscles. This study is commonly used to evaluate the electrical activity generated by a muscle when it is subjected to loads or stressing.

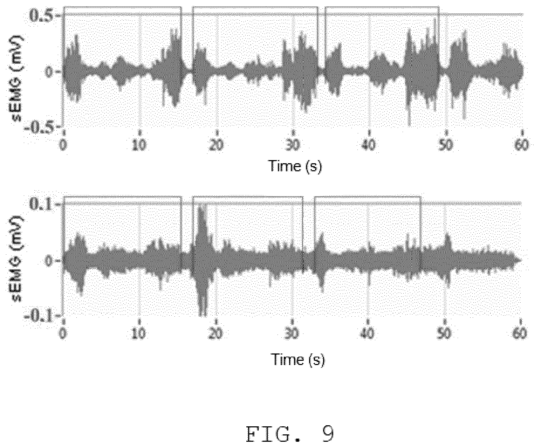

The results of this study (Figure 9) support the data obtained in the movement analysis. In the graph of Figure 9, the upper part represents the results for the tool of the prior art and the lower part represents the results for the tool according to the present invention. This study allowed showing that during a simulated standard test, the muscular activity generated with the tool of the prior art was, on average, 5 times greater than that generated with the tool according to the present invention, in the main muscles involved in movements of the arms during this practice (deltoid, bicep, trapezius, and palmaris longus muscles). This data clearly shows that, in comparative terms, these muscle groups can become fatigued sooner with conventional tools than they will with the integration of an articulated element (ball joint) according to the present invention.

Therefore, the surgical tool according to the present invention allows increasing the time the surgeon can be in a position that is the most comfortable for them, and it therefore reduces the fatigue and effort to which the surgeon is subjected. Furthermore, it reduces the muscular energy the surgeon has to use during the surgical intervention.

Another advantage of the present invention is that as a result of its particular design, the contact surface in the joint of the ball-socket type is very small, so friction during rotation of one hemisphere with respect to the other is reduced, thus optimising the operation of the surgical tool.

Furthermore, due to the fact that both the inside of the gripping clamp (28) and the base hemisphere (22) have spherical surfaces concentric to one another, the distance between both surfaces in the unlocking position is always the same, so this enables the tool being locked in any of the working angles in which it is found, thereby maximising effectiveness of the tool.

Another advantage of the present invention is that the "centre of precision" of the surgical tool is located at the centre of a sphere located in laparoscopic practice in the palm of the hand. Due to the intrinsic features of the ball joint, at the centre of same there is formed a point around which all the elements that allow actuation of the tool turn. This means that control of this point leads to precise control of the tool, for which reason said point is referred to in this specification as "centre of precision". This simple change presents the tool as an extension of the body of the surgeon, continuing the dynamics the different sections of their arm follow (the forearm is aligned with the arm, the wrist with the forearm, the hand with the wrist and the tool following the same principle).

In the case of tools from the prior art, the handle is not spherical, which means that instead of a "centre of precision", there is a central (longitudinal) handle actuation axis forming a specific angle with the longitudinal axis of the stylet, and with the wrist of the surgeon. The change in position of the tool thereby causes a movement in the surgeon's hand and/or arm which must be absorbed by the wrist.

All of this means that the surgeon's dexterity will be highly influenced, and despite the immense skills of the surgeons, it even considerably increases the chance of committing mistakes and causing injuries in the body of the patient.

Although the present invention has been described in reference to a preferred embodiment thereof, one skilled in the art may conceive of changes and modifications that do not depart from the scope of the attached claims.

## Claims

1. A surgical tool comprising:
- a stylet (12);
- a tip (10) of the surgical tool at a distal end of the stylet (12); and
- a handle (14) at a proximal end of the stylet (12), the handle (14) comprising means for actuating the tip (10) of the tool, said handle (14) being formed by a ball joint;
wherein the ball joint comprises:
- a base hemisphere (22) coupled such that it is solidly connected to an outer sheath (18) of the stylet (12);
- a central hemisphere (40) solidly connected to the base hemisphere (22) and located at a centre thereof;
- a gripping clamp (28) with a spherical inner surface concentric with the base hemisphere (22);
- a rotation hemisphere (20) solidly connected to the gripping clamp (28) and located at a centre of the inner surface thereof, forming a joint of the ball-socket type together with the central hemisphere (40); and
- locking means which allow a locking position to be adopted, in which relative movement between the gripping clamp (28) and the base hemisphere (22) is prevented, and an unlocking position, in which relative movement between the gripping clamp (28) and the base hemisphere (22) is permitted, wherein said locking means are formed by the gripping clamp (28) itself, a locking area (38) of which is formed by the spherical inner surface thereof, which can move until making contact with the base hemisphere (22) by means of pressure on sides of the gripping clamp (28).

2. The surgical tool according to claim 1, **characterised in that** the inner surface of the gripping clamp (28) is located facing the base hemisphere (22).

3. The surgical tool according to any of the preceding claims, **characterised in that** the locking area (38) consists of a pair of locking projections arranged in a space between the gripping clamp (28) and the base hemisphere (22) on opposite sides with respect to a central axis defined by a longitudinal axis of the stylet (12).

4. The surgical tool according to any of the preceding claims, **characterised in that** the means for actuating comprise:
- a trigger (30) connected to an actuation lever (32) in turn connected to a proximal end of a cable (34), a distal end of said cable (34) being connected to a proximal end of an internal rod (16) of the stylet (12) surrounded by an outer sheath (18) of the stylet (12); and
- elastic return means,
such that actuation of the trigger (30) is transmitted through the cable (34) and the internal rod (16) to the tip (10) of the tool, causing it to transition from a first position to a second position, and the elastic return means return the tip (10) of the tool from the second position to the first position in the absence of any action on said trigger (30).

5. The surgical tool according to claim 4, **characterised in that** the outer sheath (18) and the internal rod (16) are coupled to the base hemisphere (22) and to the cable (34), respectively, according to removable attachment means, selected from the group comprising threaded attachments, stud bolts, intermediate parts, or the like.

6. The surgical tool according to any of the preceding claims, **characterised in that** the tip (10) of the tool is one selected from the group comprising tips for laparoscopy tools, tips for endoscopy tools, and tips for tools utilising high-frequency technology.

## Patentansprüche

1. Chirurgisches Instrument, umfassend:
- ein Stilett (12);
- eine Spitze (10) des chirurgischen Instruments an einem distalen Ende des Stiletts (12); und
- einen Griff (14) an einem proximalen Ende des Stiletts (12), wobei der Griff (14) Mittel zum Betätigen der Spitze (10) des Instruments umfasst, wobei der Griff (14) durch ein Kugelgelenk gebildet ist;
wobei
das Kugelgelenk umfasst:
- eine Basishalbkugel (22), die derart gekoppelt ist, dass sie fest mit einem Außenmantel (18) des Stiletts (12) verbunden ist;
- eine Zentralhalbkugel (40), die fest mit der Basishalbkugel (22) verbunden ist und sich in Mitte davon befindet;
- eine Greifklemme (28) mit einer kugelförmigen Innenfläche, die konzentrisch zur Basishalbkugel (22) ist;
- eine Rotationshalbkugel (20), die fest mit der Greifklemme (28) verbunden und sich in der Mitte der Innenfläche davon befindet und zusammen mit der Zentralhalbkugel (40) ein Gelenk vom Typ Kugelpfanne bildet; und
- Verriegelungsmittel, die es erlauben, eine Verriegelungsposition einzunehmen, in der eine Relativbewegung zwischen der Greifklemme (28) und der Basishalbkugel (22) verhindert wird, und eine Entriegelungsposition, in der eine Relativbewegung zwischen der Greifklemme (28) und der Basishalbkugel (22) zugelassen wird, wobei die Verriegelungsmittel durch die Greifklemme (28) selbst gebildet werden, von der ein Verriegelungsbereich (38) durch die kugelförmige Innenfläche gebildet wird, die sich durch Druck auf Seiten der Greifklemme (28) bis zum Kontakt mit der Basishalbkugel (22) bewegen kann.

2. Chirurgisches Instrument nach Anspruch 1, **dadurch gekennzeichnet, dass** die Innenfläche der Greifklemme (28) der Basishalbkugel (22) zugewandt ist.

3. Chirurgisches Instrument nach einem der vorhergehenden Ansprüche, **dadurch gekennzeichnet, dass** der Verriegelungsbereich (38) aus einem Paar von Verriegelungsvorsprüngen besteht, die in einem Raum zwischen der Greifklemme (28) und der Basishalbkugel (22) auf gegenüberliegenden Seiten in Bezug auf eine Zentralachse angeordnet sind, die durch eine Längsachse des Stiletts (12) definiert ist.

4. Chirurgisches Instrument nach einem der vorhergehenden Ansprüche, **dadurch gekennzeichnet, dass** die Mittel zum Betätigen umfassen:
- einen Auslöser (30), der mit einem Betätigungshebel (32) verbunden ist, der wiederum mit einem proximalen Ende eines Kabels (34) verbunden ist, wobei ein distales Ende des Kabels (34) mit einem proximalen Ende einer inneren Stange (16) des Stiletts (12) verbunden ist, die von einem Außenmantel (18) des Stiletts (12) umgeben ist; und
- elastisches Rückstellmittel,
derart, dass die Betätigung des Auslösers (30) über das Kabel (34) und die innere Stange (16) auf die Spitze (10) des Instruments übertragen wird, wodurch diese von einer ersten Position in eine zweite Position übergeht, und die elastischen Rückstellmittel die Spitze (10) des Instruments von der zweiten Position in die erste Position zurückstellen, wenn keine Betätigung des Auslösers (30) erfolgt.

5. Chirurgisches Instrument nach Anspruch 4, **dadurch gekennzeichnet, dass** der Außenmantel (18) und die innere Stange (16) mit der Basishalbkugel (22) bzw. mit dem Kabel (34) über abnehmbare Befestigungsmittel verbunden sind, die aus der Gruppe ausgewählt sind, die Gewindebefestigungen, Stehbolzen, Zwischenteile oder dergleichen umfasst.

6. Chirurgisches Instrument nach einem der vorhergehenden Ansprüche, **dadurch gekennzeichnet, dass** die Spitze (10) des Instruments aus der Gruppe ausgewählt ist, die Spitzen für Laparoskopieinstrumente, Spitzen für Endoskopieinstrumente und Spitzen für Instrumente, die Hochfrequenztechnologie verwenden, umfasst.

## Revendications

1. Instrument chirurgical comprenant :
- un stylet (12) ;
- une pointe (10) de l'instrument chirurgical à une extrémité distale du stylet (12) ; et
- une poignée (14) à une extrémité proximale du stylet (12), la poignée (14) comprenant des moyens d'actionnement de la pointe (10) de l'instrument, ladite poignée (14) étant formée par un joint à rotule ;
dans lequel
le joint à rotule comprend :
- un hémisphère de base (22) accouplé de telle sorte qu'il soit solidement raccordé à une gaine externe (18) du stylet (12) ;
- un hémisphère central (40) raccordé solidement à l'hémisphère de base (22) et situé au centre de celui-ci ;
- une pince de préhension (28) avec une surface interne sphérique concentrique à l'hémisphère de base (22) ;
- un hémisphère de rotation (20) raccordé solidement à la pince de préhension (28) et situé au centre de la surface interne de celle-ci, formant un joint du type sphérique avec l'hémisphère central (40) ; et
- des moyens de verrouillage qui permettent d'adopter une position de verrouillage, dans laquelle le mouvement relatif entre la pince de préhension (28) et l'hémisphère de base (22) est empêché et une position de déverrouillage, dans laquelle le mouvement relatif entre la pince de préhension (28) et l'hémisphère de base (22) est autorisé, dans lequel lesdits moyens de verrouillage sont formés par la pince de préhension (28) elle-même, dont une zone de verrouillage (38) est formée par la surface interne sphérique de celle-ci, qui peut se déplacer jusqu'à entrer en contact avec l'hémisphère de base (22) au moyen d'une pression sur les côtés de la pince de préhension (28).

2. Instrument chirurgical selon la revendication 1, **caractérisé en ce que** la surface interne de la pince de préhension (28) est située en regard de l'hémisphère de base (22).

3. Instrument chirurgical selon l'une quelconque des revendications précédentes, **caractérisé en ce que** la zone de verrouillage (38) se compose d'une paire de saillies de verrouillage agencées dans un espace entre la pince de préhension (28) et l'hémisphère de base (22) sur des côtés opposés par rapport à un axe central défini par un axe longitudinal du stylet (12).

4. Instrument chirurgical selon l'une quelconque des revendications précédentes, **caractérisé en ce que** les moyens d'actionnement comprennent :
- une gâchette (30) raccordée à un levier d'actionnement (32) raccordé à son tour à une extrémité proximale d'un câble (34), une extrémité distale dudit câble (34) étant raccordée à une extrémité proximale d'une tige interne (16) du stylet (12) entourée par une gaine externe (18) du stylet (12) ; et
- des moyens de rappel élastique,
de telle sorte que l'actionnement de la gâchette (30) soit transmis par le biais du câble (34) et de la tige interne (16) à la pointe (10) de l'instrument, le faisant passer d'une première position à une seconde position et les moyens de rappel élastique ramènent la pointe (10) de l'instrument de la seconde position à la première position en l'absence de toute action sur ladite gâchette (30).

5. Instrument chirurgical selon la revendication 4, **caractérisé en ce que** la gaine externe (18) et la tige interne (16) sont accouplées à l'hémisphère de base (22) et au câble (34), respectivement, selon des moyens de fixation amovibles, choisis dans le groupe comprenant les fixations filetées, les goujons, les pièces intermédiaires ou similaires.

6. Instrument chirurgical selon l'une quelconque des revendications précédentes, **caractérisé en ce que** la pointe (10) de l'instrument est choisie dans le groupe comprenant les pointes pour instruments de laparoscopie, les pointes pour instruments d'endoscopie et les pointes pour instruments utilisant la technologie à haute fréquence.
